(19) Europäisches Patentamt
European Patent Office
Office européen des brevets

(11) Veröffentlichungsnummer: **0 003 361**
**B1**

(12) # EUROPÄISCHE PATENTSCHRIFT

(45) Veröffentlichungstag der Patentschrift: **16.06.82**

(21) Anmeldenummer: **79100231.4**

(22) Anmeldetag: **26.01.79**

(51) Int. Cl.³: **C 07 C 33/02,**
C 07 C 33/04,
C 07 C 31/02,
C 07 C 69/07,
C 07 C 69/145,
C 07 C 69/24, C 11 B 9/00,
C 07 C 69/06, C 07 C 69/14
//A61K7/46, C09D7/12,
C09J3/00

(54) **3,5,5-Trimethyl-hexan-1-ole und deren Ester sowie die Herstellung der neuen Verbindungen und deren Verwendung als Duftstoffe.**

(30) Priorität: **01.02.78 DE 2804167**

(43) Veröffentlichungstag der Anmeldung:
**08.08.79 Patentblatt 79/16**

(45) Bekanntmachung des Hinweises auf die Patenterteilung:
**16.06.82 Patentblatt 82/24**

(84) Benannte Vertragsstaaten:
**CH DE FR GB NL**

(56) Entgegenhaltungen:
DE - B - 1 044 326
DE - C - 544 388
US - A - 3 855 322
US - A - 4 002 647

(73) Patentinhaber: **BASF Aktiengesellschaft**
**Carl-Bosch-Strasse 38**
**D-6700 Ludwigshafen (DE)**

(72) Erfinder: **Nohe, Heinz, Dr.**
**Ruppertsberger Strasse 7b**
**D-6701 Meckenheim (DE)**
Erfinder: **Wenisch, Franz, Dr.**
**Anselm-Feuerbach-Strasse 14**
**D-6710 Frankenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 003 361 B1

# 3,5,5-Trimethyl-hexan-1-ole und deren Ester sowie die Herstellung der neuen Verbindungen und deren Verwendung als Duftstoffe

Die vorliegende Erfindung betrifft 3,5,5-Trimethyl-hexan-1-ole und deren Ester der allgemeinen Formel I

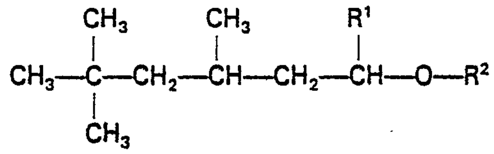

I,

in der

$R^1$ einen Alkylrest mit 2 oder 3 C-Atomen, einen olefinisch ungesättigten aliphatischen Rest mit 2 bis 4 C-Atomen, vorzugsweise 2 oder 3 C-Atomen, den Äthinyl- oder den Propargylrest bedeutet und in der

$R^2$ für Wasserstoff oder eine Acylgruppe mit 1 bis 4 C-Atomen steht.

Ferner betrifft die Erfindung die Verwendung der neuen Verbindungen der Formel I als Duftstoffe in kosmetischen Zubereitungen aller Art sowie in Wasch- und Reinigungsmitteln oder als Duftverbesserer für Leime, Anstrichmittel und ähnliche Erzeugnisse. Besonders ansprechende Duftnoten besitzen 5,7,7-Trimethyl-1-octen-3-ol und 6,8,8-Trimethyl-1-nonen-4-ol, was ziemlich überraschend ist, da das 3,5,5-Trimethylhexanol einen mehr "chemischen" und nicht sehr angenehmen Duft aufweist (vgl. Steefen Arctander: "Perfume and Flavor Chemicals" 1969, Hoffensbergske Etablissement Kopenhagen Nr. 3007).

Es wurde gefunden, daß man diese als Duftstoffe wertvollen Verbindungen in an sich bekannter Weise erhält, wenn man 3,5,5-Trimethylhexan-1-al (II) mit einer Grignard-Verbindung $R^1$-Mg-Halogen (III) umsetzt und das hierbei entstehende 3,5,5-Trimethyl-hexan-1-ol gegebenenfalls mit einer niederen aliphatischen Carbonsäure, dem entsprechenden Carbonsäurechlorid oder Carbonsäureanhydrid umsetzt.

Die Ausgangsverbindung (II) ist bekannt und durch Hydroformylierung von 2,4,4-Trimethyl-pent-1-en ("Diisobutylen") erhältlich.

Als Grignard-Verbindungen (III) eignen sich im Hinblick auf die Verfahrensprodukte (I) besonders diejenigen, in denen $R^1$ eine Äthyl-, Isopropyl-, Isopropenyl-, Vinyl-, Allylgruppe, Äthinyl- oder Propargylgruppe ist, insbesondere Grignard-Verbindungen (III), in denen $R^1$ eine Vinyl- oder Allylgruppe bedeutet.

Die Grignard-Verbindungen III werden in üblicher Weise erhalten, indem man die Halogenverbindungen $R^1$-Halogen in inerten Lösungsmitteln mit Magnesium umsetzt. Von den Halogenverbindungen werden die Chloride, Bromide und Jodide, insbesondere die Chloride und Bromide eingesetzt.

Zweckmäßig verwendet man die Grignard-Verbindung in einer Menge von etwa 0,8 bis 1,5 Mol pro Mol des Aldehyds der Formel II.

Als Lösungsmittel eignen sich insbesondere Diäthyläther oder Tetrahydrofuran. Jedoch können auch höhere Äther, wie Diisopropyläther, Dibutyläther, Diamyläther oder Anisol sowie tertiäre Amine, wie Pyridin und Chinolin als Lösungsmittel verwendet werden.

Zur Durchführung des Verfahrens geht man zweckmäßig so vor, daß man den Aldehyd oder eine Lösung des Aldehyds in dem gewählten Lösungsmittel langsam zu der Lösung des Alkylmagnesiumhalogenids zufügt und das Reaktionsgemisch noch etwa 1/2 bis 5 Stunden, vorzugsweise 1 bis 2 Stunden, nachreagieren läßt.

Die Reaktion kann bei Temperaturen von $-10$ bis $+50°C$ durchgeführt werden. Bevorzugt arbeitet man jedoch bei Raumtemperatur, da hierbei die Nebenproduktbildung besonders gering ist.

Voraussetzung für das Gelingen der Reaktion ist die Reinheit und absolute Trockenheit der Reaktionspartner sowie des Lösungsmittels.

Die Aufarbeitung des Reaktionsgemisches erfolgt wie üblich durch Hydrolyse mit Wasser bzw. verdünnter Mineralsäure, Abtrennung der organischen Phase sowie Extraktion der wäßrigen Phase mit dem für die Reaktionsführung gewählten Lösungsmittel.

Bei Verwendung des wasserlöslichen THF als Lösungsmittel versetzt man das Reaktionsgemisch mit Wasser, saugt das ausfallende basische Magnesiumchlorid ab, wäscht mit THF nach und fraktioniert die vereinigten THF-Phasen.

Zur Herstellung der Ester der Formel I setzt man den Alkohol I in üblicher Weise mit einer niederen Carbonsäure der Formel $R^2$—COOH oder mit dem entsprechenden Säurechlorid der Formel $R^2$—COCl oder dem entsprechenden Säureanhydrid um.

Die Aufarbeitung des Reaktionsansatzes aus dem Alkohol I und HCOOH gestaltet sich besonders einfach, da die gebildeten Formiate hierin nicht löslich sind und einfach abgesaugt werden können. Bei Umsetzen des Alkohols I mit Essigsäure oder Acetanhydrid destilliert man nach der Umsetzung vorteilhaft zunächst die Essigsäure bzw. das Acetanhydrid ab, wäscht das erhaltene Rohprodukt mit Wasser und destilliert gegebenenfalls in Gegenwart alkalischer Mittel wie $CaCO_3$ oder $NaHCO_3$.

Die Umsetzung mit einem Säurechlorid oder einem Säureanhydrid erfolgt mit Vorteil so, daß man das Säurechlorid oder das Säureanhydrid bei Temperaturen von etwa 0 bis 80°C langsam direkt in das bei der Umsetzung des Aldehyds II mit der Grignard-Verbindung III gebildete Reaktionsgemisch einbringt und erst das hierbei erhaltene Reaktionsgemisch auf übliche Weise durch Eingießen in Eiswasser, Abtrennen der organischen Phase, Trocknen und Fraktionieren aufarbeitet.

Die Umsetzung mit einem Säurechlorid kann aber auch in üblicher Weise in Gegenwart von Pyridin durchgeführt werden.

Das Säurechlorid und das Säureanhydrid verwendet man im allgemeinen in Mengen von 1 bis 1,5 Mol pro Mol des Aldehyds II.

Die Verfahrensprodukte (I) sind Flüssigkeiten mit z.T. sehr interessanten Geruchsnoten.

Die erfindungsgemäßen Duftstoffe können für die eingangs genannten Zwecke verwendet werden, und zwar entweder in reiner Form oder in Mischung mit anderen Duftstoffen. Für die Verwendung in reiner Form eignen sich besonders diejenigen Verbindungen, in denen die Reste $R^1$ und $R^3$ je bis zu 3 C-Atomen enthalten, während die übrigen Verbindungen mehr zur Abrundung und zur Erhöhung der Permanenz in vorwiegend durch andere Komponenten bestimmten Kompositionen dienen. Die Mengen dieser Duftstoffe entsprechen denen von bekannten Geruchsträgern. So verwendet man beispielsweise das 3,7,7-Trimethyl-1-octen-3-ol bzw. dessen Acetat bei der Seifenherstellung derart, daß man der Seife bis zu etwa 3% einer Duftstoffkomposition zusetzt, die bis etwa 5% Trimethyloctenol und/oder dessen Acetat enthält. Bei der Herstellung von Rasierwässern setzt man im allgemeinen bis zu 1% einer Duftstoffkomposition zu, die maximal bis zu 10% 3,7,7-Trimethyl-1-octen-3-ol und/oder sein Acetat neben anderen Duftstoffen enthält. Bei der Herstellung von Hautcremes setzt man der Creme im allgemeinen bis zu 0,5% einer Duftstoffkomposition zu, die bis zu 10% 3,7,7-Trimethyl-1-octen-3-ol und/oder dessen Acetat enthält. Bei der Herstellung, von Waschpulvern setzt man dem Waschpulver etwa 0,15% einer Duftstoffkomposition zu, die bis etwa 5% 3,7,7-Trimethyl-1-octen-3-ol enthält. Besondere Bedeutung wegen ihrer besonders ansprechenden Duftnoten besitzen das 3,7,7-Trimethyl-1-octen-3-ol, dessen Acetat sowie das 4,8,8-Trimethyl-1-nonen-4-ol.

Beispiel 1 bis 9

Je 284 g (2 Mol) 3,5,5-Trimethyl-hexan-1-al wurden bei 20°C im Laufe von 60 Minuten in eine Lösung bestehend aus 1 660 ml Tetrahydrofuran (THF) und 2,5 Mol einer wie üblich hergestellten Grignard-Verbindung $R^1$—Mg—Cl eingetropft. Nach beendeter Reaktion wurde das Gemisch mit 300 ml Wasser versetzt, wobei darauf geachtet wurde, daß die Temperatur 35°C nicht überstieg. Das ausgefallene basische Magnesiumchlorid wurde abgesaugt und mit je 100 ml Tetrahydrofuran gewaschen. Die organische Phase wurde mit den Waschflüssigkeiten vereinigt und hieraus bei 64°C das THF zusammen mit Wasser abdestilliert. Das Rohprodukt wurde anschließend unter stark vermindertem Druck destilliert.

In der folgenden Tabelle sind die Ergebnisse der Versuche sowie die Charakteristika der Verfahrensprodukte zusammengestellt.

* bezogen auf eingesetztes 3,5,5-Trimethyl-hexan-1-al.
** erhalten durch Hydrieren der entsprechenden Verbindung der Formel I mit $R^1 = -CH{=}CH_2$ an $PtO_2$ bei 20°C; Ausbeuteangaben bez. auf Ausgangsverbind.

| Beispiel | $R^1$ | $R^2$ | Ausbeuten* % | Siedepunkt °C/mbar | $n_D^{20}$ | Duftcharakter |
|---|---|---|---|---|---|---|
| 1 | $-CH{=}CH_2$ | $-H$ | 84 | 83—85°/15 | 1,4430 | intensiv Enzian-wurzel-Wachholderbeer |
| 2 | $-CH{=}CH_2$ | $-CO-H$ | 64 | 151°/100 | 1,4402 | fruchtig, würzig |
| 3 | $-CH{=}CH_2$ | $-CO-CH_3$ | 67 | 113°/16 | 1,4318 | intensiv fruchtig, Wachholderbeer |
| 4 | $-CH{=}CH_2$ | $-CO-CH_2-CH_3$ | 69 | 68—70°/0,5 | 1,4343 | Kartoffelkeller, erdig |
| 5 | $-CH{=}CH_2$ | $-CO-CH_2-CH_2-CH_3$ | 66 | 67—68°/0,3 | 1,4353 | säuerlich, Bierhefe |
| 6** | $-CH_2-CH_3$ | $-H$ | 90 | 97°/14 | 1,4398 | intensiv fruchtig, süß, birnenartig |
| 7** | $-CH_2-CH_3$ | $-CO-CH_3$ | 92 | 99°/16 | 1,4243 | heuartig, coumarin-artig |
| 8 | $CH_3$<br>\|<br>$-CH-CH_3$ | $-H$ | 57 | 64°/0,4 | 1,4398 | schweißartig, schwach Wachholder |
| 9 | $-CH_2-CH{=}CH_2$ | $-H$ | 77 | 51°/0,1 | 1,4465 | intensiv Holunder, Wachholder |

**Patentansprüche**

1. 3,5,5-Trimethyl-hexan-1-ole und deren Ester der allgemeinen Formel I

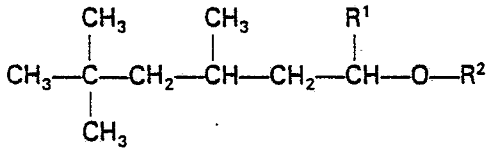

I,

in der

$R^1$ einen Alkylrest mit 2 oder 3 C-Atomen, einen olefinisch ungesättigten aliphatischen Rest mit 2 bis 4 C-Atomen, den Äthinyl- oder den Propargylrest bedeutet und in der

$R^2$ für Wasserstoff oder einen Acylgruppe mit 1 bis 4 C-Atomen steht.

2. 5,7,7-Trimethyl-1-octen-3-ol.

3. 5,7,7-Trimethyl-1-octen-3-ol-acetat.

4. 6,8,8-Trimethyl-1-nonen-4-ol.

5. Verfahren zur Herstellung der 3,5,5-Trimethylhexan-1-ole und deren Ester der allgemeinen Formel I gemäß Anspruch 1, dadurch gekennzeichnet, daß man 3,5,5-Trimethyl-hexan-1-al (II) in an sich bekannter Weise mit einer Grignard-Verbindung $R^1$-Mg-Halogen (III; Halogen=Cl, Br oder J) umsetzt und das hierbei entstehende 3,5,5-Trimethyl-hexan-1-ol gegebenenfalls mit einer niederen Carbonsäure, dem entsprechenden Carbonsäurechlorid oder Carbonsäureanhydrid umsetzt.

6. Verwendung der 3,5,5-Trimethyl-hexan-1-ole und deren Ester der allgemeinen Formel I gemäß den Ansprüchen 1 bis 5 als Duftstoffe in kosmetischen Zubereitungen, in Wasch- und Reinigungsmitteln sowie als Duftverbesserer für Leime und Anstriche.

**Claims**

1. 3,5,5-Trimethyl-hexan-1-ols and their esters of the general formula I

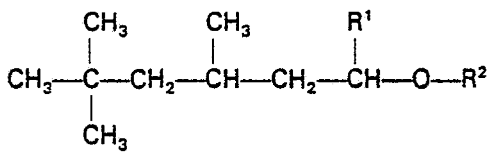

I,

where

$R^1$ denots alkyl of 2 or 3 carbon atoms, an olefinically unsaturated aliphatic radical of from 2 to 4 carbon atoms, ethynyl or propargyl, and

$R^2$ denotes hydrogen or an acyl group of from 1 to 4 carbon atoms.

2. 5,7,7-Trimethyl-1-octen-3-ol.

3. 5,7,7-Trimethyl-1-octen-3-ol acetate.

4. 6,8,8-Trimethyl-1-nonen-4-ol.

5. A process for the production of 3,5,5-trimethyl-hexan-1-ols and their esters of the general formula I as claimed in claim 1, characterized in that 3,5,5-trimethyl-hexan-1-al (II) is reacted, in conventional manner, with a Grignard compound $R^1$-Mg-halogen (III; halogen=Cl, Br or I) and, if desired, reacting the resulting 3,5,5-trimethyl-hexan-1-ol with a lower carboxylic acid or the corresponding carboxylic acid chloride or carboxylic acid anhydride.

6. The use of 3,5,5-trimethyl-hexan-1-ols and their esters of the general formula I as claimed in any of claims 1 to 5 as fragrance materials in cosmetic preparations, detergents and cleaning agents and as odour improvers for adhesives and paints.

**Revendications**

1. 3,5,5-Triméthyl-hexane-1-ols et leurs esters de formule générale I

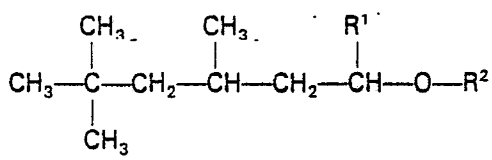

I,

dans laquelle

$R^1$ représente un groupe alkyle en C2—C3, un radical aliphatique à insaturation oléfinique en C2—C4, le groupe éthynyle ou le groupe propargyle, et

$R^2$ représente l'hydrogène ou un groupe acyle en C1—C4.

2. Le 5,7,7-triméthyl-1-octène-3-ol.

3. L'acétate du 5,7,7-triméthyl-1-octène-3-ol.

4. Le 6,8,8-triméthyl-1-nonène-4-ol.

5. Procédé de préparation des 3,5,5-triméthyl-hexane-1-ols et de leurs esters de formule générale I selon la revendication 1, caractérisé en ce que l'on fait réagir de manière connue en soi le 3,5,5-triméthyl-hexane-1-al (II) de manière connue en soi avec un composé de Grignard $R^1$-Mg-halogène (III; halogène=Cl, Br ou I) la réaction donnant un 3,5,5-triméthyl-hexane-1-ol qu'on fait réagir le cas échéant avec un acide carboxylique inférieur, le chlorure d'acide ou l'anhydride correspondant.

6. Utilisation des 3,5,5-triméthyl-hexane-1-ols et de leurs esters de formule générale I selon les revendications 1 à 5 en tant que substances odorantes dans des compositions cosmétiques, des produits de lavage et de nettoyage et comme correcteurs d'odeurs dans des colles et produits de revêtement.